(19)

Europäisches
Patentamt
European
Patent Office
Office européen
des brevets

(11) **EP 4 338 742 A1**

(12) **EUROPEAN PATENT APPLICATION**
published in accordance with Art. 153(4) EPC

(43) Date of publication:
**20.03.2024 Bulletin 2024/12**

(21) Application number: **22819662.2**

(22) Date of filing: **11.06.2022**

(51) International Patent Classification (IPC):
***A61K 31/57*** (2006.01)    ***A61K 45/06*** (2006.01)
***A61P 35/00*** (2006.01)

(52) Cooperative Patent Classification (CPC):
**A61K 31/57; A61K 39/395; A61K 45/06;**
**A61P 35/00**

(86) International application number:
**PCT/CN2022/098284**

(87) International publication number:
**WO 2022/258066 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **11.06.2021   CN 202110655991**
            **10.06.2022   CN 202210651649**

(71) Applicant: **Shenzhen Evergreen Therapeutics Co., Ltd.**
**Shenzhen, Guangdong 518038 (CN)**

(72) Inventors:
• **DU, Tao Tom**
  **Shenzhen, Guangdong 518038 (CN)**
• **HU, Tao**
  **Shenzhen, Guangdong 518038 (CN)**
• **DU, Xin**
  **Shenzhen, Guangdong 518038 (CN)**
• **ZHANG, Yuqian**
  **Shenzhen, Guangdong 518038 (CN)**

(74) Representative: **Groth & Co. KB**
**P.O. Box 6107**
**102 32 Stockholm (SE)**

(54) **APPLICATION OF HYDROXYPROGESTERONE CAPROATE IN ENHANCING TUMOR TREATMENT EFFECT**

(57)    A method for co-administrating hydroxyprogesterone caproate, tyrosine kinase inhibitor, and/or anti-PD-I/PD-LI an-I tibodies for tumor treatment. The present application also provides a corresponding pharmaceutical use and a pharmaceutical product. Co-administration with hydroxyprogesterone caproate can further improve the therapeutic effect of lenvatinib and anti-PD-I/PD-LI antibodies against tumors.

Figure 7

EP 4 338 742 A1

**Description**

**Technical Field**

This application belongs to the field of tumor treatment. Specifically, it provides a method for inhibiting tumors using hydroxyprogesterone caproate along with receptor tyrosine kinase inhibitors and/or anti-PD-1/PD-L1 antibodies.

**Background**

Unlike traditional cancer treatment methods such as surgical resection, chemotherapy, and radiation therapy, immunotherapy for tumors primarily aims to eliminate cancer cells and inhibit tumor growth by activating the body's own immune system. With the continuous expansion and deepening of tumor immunology-related research, various clinical immunotherapeutic approaches for tumors have flourished. These encompass non-specific immune stimulants, adoptive cell therapy, anti-tumor monoclonal antibodies, and tumor-specific vaccines. Immunotherapy, characterized by its high specificity and minimal harm to the body, has gained increasing significance in modern cancer treatments. Among these approaches, immune checkpoint inhibitors have seen the most extensive and comprehensive clinical research.

Immune checkpoints are molecules involved in regulating the intensity of immune responses within the tumor immune signaling pathway. They help maintain self-immune tolerance and are involved in tumor immune escape. Immune checkpoint inhibitors, a class of monoclonal antibody drugs developed against specific immune checkpoints, activate tumor-specific T cells and rebuild the immune system to induce the killing of tumor cells. Currently, among the most crucial immune checkpoints in tumor immunotherapy are cytotoxic T-lymphocyte-associated antigen 4 (CTLA-4), programmed cell death protein 1 (PD-1), programmed death-ligand 1 (PD-L1), lymphocyte activation gene 3 (LAG-3), T-cell immunoglobulin and mucin-domain containing-3 (TIM-3), T-cell immunoreceptor with Ig and ITIM domains (TIGIT), and V-domain Ig suppressor of T-cell activation (VISTA). Notably, monoclonal antibodies targeting CTLA-4, PD-1, and PD-L1 have received approval from the US FDA for tumor therapy. For instance, anti-CTLA-4 antibody ipilimumab was approved for melanoma treatment in 2011, while anti-PD-1 antibodies pembrolizumab and nivolumab were approved for melanoma, non-small cell lung cancer, and head and neck squamous cell carcinoma therapy in 2014. Additionally, anti-PD-L1 antibody atezolizumab was approved for bladder cancer and non-small cell lung cancer treatment in 2016. Several monoclonal antibodies targeting these immune checkpoints are either approved for market use or are undergoing clinical trials for tumor therapy.

However, it's worth noting that while the body's immune system plays its role in immune function, tumor cells adapt by altering their surface antigens and modifying the tumor microenvironment to evade recognition and surveillance by the immune system, thereby reducing the effectiveness of immune therapy. For instance, reports indicate that the clinical response rate of anti-PD-1 antibodies is only around 20%, highlighting immune escape as one of the challenges in clinical tumor immunotherapy. Studies suggest that mechanisms leading to tumor immune escape include: 1) tumor cell-related factors such as abnormal tumor cell surface antigen expression, abnormal expression of receptors like Fas, and secretion of immune-inhibitory factors like transforming growth factor-beta (TGF-β) and interleukin-10 (IL-10); 2) immune cell-related factors involving immunosuppressive effects of regulatory T cells, phenotypic conversion of natural killer (NK) cells, tumor-associated macrophage polarization, and dysfunction of dendritic cells (DCs); 3) immune suppression-related factors in the tumor microenvironment like vascular endothelial growth factor (VEGF). Changes in tumor cells, immune cells, and the tumor microenvironment are closely linked and interact, leading to immune escape of tumor cells and reduced efficacy of immune therapy in clinical settings.

To address immune escape and immune tolerance issues of tumor cells, combination therapies involving different immune checkpoint inhibitors, combined chemotherapy and immunotherapy, among others, undoubtedly exhibit greater potential and efficacy in clinical tumor treatments. Clinical data has shown that the combination therapy of anti-PD-1 antibody nivolumab and anti-CTLA-4 antibody ipilimumab has demonstrated superior therapeutic effects in melanoma, advanced renal cell carcinoma, colorectal cancer, breast cancer, non-small cell lung cancer, and esophageal cancer treatments. In May 2020, the US FDA also approved the combination therapy of nivolumab and ipilimumab for first-line treatment in non-small cell lung cancer patients with tumor PD-L1 expression (≥ 1%) and without epidermal growth factor receptor (EGFR) or anaplastic lymphoma kinase (ALK) gene mutations. Given the enhanced therapeutic potential of combination therapy, there exists a significant need in this field for drugs capable of augmenting the efficacy of tumor immunotherapy.

**Summary of the Invention**

On one hand, the present application provides a method for inhibiting tumors in a subject, characterized in that hydroxyprogesterone caproate, as well as a receptor tyrosine kinase inhibitor and/or anti-PD-1/PD-L1 antibodies, are administered to the subj ect.

On the other hand, the present application provides the use of hydroxyprogesterone caproate, as well as a receptor tyrosine kinase inhibitor and/or anti-PD-1/PD-L1 antibodies, in the preparation of drugs for treating tumors.

Furthermore, the present application provides a drug for treating tumors, characterized by containing hydroxyprogesterone caproate, as well as a receptor tyrosine kinase inhibitor and/or anti-PD-1/PD-L1 antibodies.

Moreover, the tumors include endometrial cancer, intestinal tumors, hepatocellular carcinoma, melanoma, non-small cell lung cancer, renal cell carcinoma, head and neck squamous cell carcinoma, urothelial carcinoma, biliary tract cancer, colorectal cancer, gastric cancer, glioblastoma, ovarian cancer, pancreatic cancer, or triple-negative breast cancer.

Furthermore, the intestinal tumors are colorectal tumors, preferably tumors formed by CT26 tumor cells.

The dosage of hydroxyprogesterone caproate is 1-100mg/kg, preferably 5-80 mg/kg, more preferably 10-70 mg/kg, further preferably 20-60 mg/kg, further preferably 20-40 mg/kg, and most preferably 20 mg/kg.

The administration frequency of hydroxyprogesterone caproate is from twice daily to once every 5 days, preferably once daily to once every 3 days, most preferably once every 2 days.

The dosage of the receptor tyrosine kinase inhibitor is 1-20 mg/kg, preferably 5-15 mg/kg, and most preferably 10 mg/kg.

The administration frequency of the receptor tyrosine kinase inhibitor is from twice daily to once every 2 days, preferably once daily.

The dosage of anti-PD-1/PD-L1 antibodies is 0.1-10 mg/kg, preferably 0.5-5 mg/kg, more preferably 1-3 mg/kg, and most preferably 2 mg/kg.

The administration frequency of anti-PD-1/PD-L1 antibodies is from twice daily to once every 6 days, preferably once daily to once every 5 days, most preferably once every 3 days.

The receptor tyrosine kinase inhibitor is Avapritinib, Capmatinib, Pemigatinib, Ripretinib, Selpercatinib, Selumetinib, Tucatinib, Entrectinib, Erdafitinib, Fedratinib, Pexidartinib, Upadacitinib, Zanubrutinib, Baricitinib, Binimetinib, Dacomitinib, Fostamatinib, Gilteritinib, Larotrectinib, Lorlatinib, Acalabrutinib, Brigatinib, Midostaurin, Neratinib, Alectinib, Cobimetinib, Lenvatinib, Osimertinib, Ceritinib, Nintedanib, Afatinib, Ibrutinib, Trametinib, Axitinib, Bosutinib, Cabozantinib, Ponatinib, Regorafenib, Tofacitinib, Crizotinib, Ruxolitinib, Vandetanib, Pazopanib, Lapatinib, Nilotinib, Dasatinib, Sunitinib, Sorafenib, Erlotinib, Gefitinib, and/or Imatinib; Lenvatinib is preferred.

The anti-PD-1/PD-L1 antibodies are 10F.9G2, RMP1-14, Pembrolizumab, Nivolumab, Cemiplimab, Atezolizumab, Avelumab, Durvalumab, Camrelizumab, Tislelizumab, Toripalimab, Sintilimab, Dostarlimab, and/or Retifanlimab.

Moreover, hydroxyprogesterone caproate, receptor tyrosine kinase inhibitors, and anti-PD-1/PD-L1 antibodies are oral formulations or injectables; the specific formulations of hydroxyprogesterone caproate, receptor tyrosine kinase inhibitors, and anti-PD-1/PD-L1 antibodies can be the same or different.

The ratio of hydroxyprogesterone caproate, receptor tyrosine kinase inhibitors, and anti-PD-1/PD-L1 antibodies in the drug is 1-100:2-20:0.1-10, preferably 20-60:5-15:0.5-5, and most preferably 20:10:2.

On the other hand, the present application provides the use of hydroxyprogesterone caproate in enhancing the effects of receptor tyrosine kinase inhibitors and/or anti-PD-1/PD-L1 antibodies.

Moreover, the present application provides the use of hydroxyprogesterone caproate in preparing drugs to enhance the effects of receptor tyrosine kinase inhibitors and/or anti-PD-1/PD-L1 antibodies.

The effects include inhibiting tumor growth, improving the regulation of the tumor's immunosuppressive microenvironment, enhancing infiltration of CD8+ T cells, and improving the regulation of tumor angiogenesis.

The tumors include endometrial cancer or intestinal tumors.

The intestinal tumors are colorectal tumors, preferably tumors formed by CT26 tumor cells.

The dosage of hydroxyprogesterone caproate is 1-100mg/kg, preferably 5-80 mg/kg, more preferably 10-70 mg/kg, further preferably 20-60 mg/kg, further preferably 20-40 mg/kg, and most preferably 20 mg/kg.

The administration frequency of hydroxyprogesterone caproate is from twice daily to once every 5 days, preferably once daily to once every 3 days, most preferably once every 2 days.

The receptor tyrosine kinase inhibitor is Lenvatinib.

The anti-PD-1/PD-L1 antibody is Pembrolizumab, Nivolumab, Cemiplimab, Atezolizumab, Avelumab, Durvalumab, Camrelizumab, Tislelizumab, Toripalimab, Sintilimab, Dostarlimab, and/or Retifanlimab.

Hydroxyprogesterone caproate is an oral formulation or an injectable.

The method of the present application can be used for therapeutic or non-therapeutic purposes, either in vivo or in vitro.

The tumors in the present application are not limited to the listed types in the application document, and other tumors can be extended for application by those skilled in the art according to relevant research. The efficacy of the drug is verified in a colon cancer model formed by CT26 tumor cells or other tumor cell lines in the application, which does not limit the tumors within these cell lines or their corresponding cancer types in the scope of protection of the application.

The drug in the present application can be used alone or in further combination with other anti-tumor drugs or therapies, including but not limited to radiotherapy, chemotherapy, targeted drugs, traditional Chinese medicine or Chinese patent drugs, and health products with anti-tumor functions.

Hydroxyprogesterone caproate in the present application is also referred to as 17-α-hydroxyprogesterone caproate, 17-HPC, 17-hydroxyprogesterone caproate, 17-hydroxyhexanoate, CAS number 630-56-8.

The receptor tyrosine kinase inhibitors in the present application include but are not limited to the listed types in the application document, and various existing commercially available and under development receptor tyrosine kinase inhibitors can be used in the application.

Lenvatinib in the present application is also known as lenvatinib, E7080 (research code).

Anti-PD-1/PD-L1 antibodies may be represented by their Chinese or English antibody names or drug names, which have equivalent effectiveness. The anti-PD-1/PD-L1 antibodies in the present application include but are not limited to the listed types in the application document, and various existing commercially available and under development anti-PD-1/PD-L1 antibodies can be used in the application.

The drugs described in the present application are not limited to a single type of drug combination. Depending on the nature of the drug and application requirements, the drugs described in the application can be a combination of several types of formulations, such as oral receptor tyrosine kinase inhibitors, hydroxyprogesterone caproate injections, and anti-PD-1/PD-L1 antibody injections.

The available formulations in the present application include but are not limited to injectables such as injections, powder needles, oral formulations such as tablets, capsules, oral solutions, throat or nasal sprays, topical formulations such as ointments, sprays, patches, etc.; preferably injectables and oral formulations. The amount of active ingredients contained in these formulations can be selected based on the dosage range described in the application and the conditions of the subject (such as weight, age, and condition). Preferably, the amount of active ingredients in a unit dosage form is a single dose or a fraction thereof.

Those skilled in the pharmaceutical field can choose suitable excipients for the drugs involved in the present application according to general knowledge in the pharmaceutical field. The types of available excipients include but are not limited to solvents, co-solvents, stabilizers, dispersants, viscosity regulators, antioxidants, sweeteners, binders, gas generators, etc.

## Brief Description of the Drawings

Figure 1 shows the change in tumor volume with combined drug treatment.

Figure 2 shows the tumor weight at the end of the combined drug experiment.

Figure 3 shows that in the CT26 colon cancer model in mice, hydroxyprogesterone caproate significantly increases the infiltration of CD4+ T cells in tumor tissue when combined with lenvatinib and anti-PD-1 antibody compared to the group treated with lenvatinib and anti-PD-1 antibody alone.

Figure 4 shows that in the CT26 colon cancer model in mice, hydroxyprogesterone caproate significantly increases the infiltration of CD8+ T cells in tumor tissue when combined with lenvatinib and anti-PD-1 antibody compared to the group treated with lenvatinib and anti-PD-1 antibody alone.

Figure 5 shows that in the CT26 colon cancer model in mice, hydroxyprogesterone caproate significantly enhances the infiltration of natural killer cells in tumor tissue when combined with lenvatinib and anti-PD-1 antibody compared to the group treated with lenvatinib and anti-PD-1 antibody alone.

Figure 6 shows that in the CT26 colon cancer model in mice, hydroxyprogesterone caproate combined with lenvatinib and anti-PD-1 antibody significantly increases the levels of TNF-α, IL-2, and IFN-γ in tumor tissue compared to the group treated with lenvatinib and anti-PD-1 antibody alone.

Figure 7 shows the tumor growth curve in BALB/c mice with Renca renal cancer cell tumor models for each treatment group.

## Detailed Description

### Example 1: Model and Drug Administration

Establishment of the BALB/c Mouse CT26 Tumor Model:

Female BALB/c mice aged 6-8 weeks with a body weight of approximately 20 grams were used. CT26 cells ($5.0 \times 10^6$) dissolved in 100 μL of PBS were subcutaneously injected into BALB/c mice. When the tumor volume reached approximately 100 mm$^3$, the experimental animals were randomly divided into control and various treatment groups for the experiment.

Lenvatinib was administered orally once daily at a dosage of 10 mg/kg. Hydroxyprogesterone caproate was administered via intraperitoneal injection (abbreviated as i.p. in the table below) every two days. Anti-PD-1 antibody (RMP1-14), diluted in PBS, was administered every three days at a dosage of 200 $\mu$g per mouse.

**Example 2: Combined Drug Effects**

| Group | Drug Administration |
|---|---|
| 1. Control group (n=10) | Control agents |
| 2. Hydroxyprogesterone Caproate (n=10) | Hydroxyprogesterone Caproate (20 mg/kg, i.p., once every two days) |
| 3. Anti-PD-1 (n=10) | Anti-PD-1 RMP1-14 (200 $\mu$g/mouse, i.p., once every three days) |
| 4. Hydroxyprogesterone Caproate & Anti-PD-1 (n=10) | Hydroxyprogesterone Caproate (20 mg/kg, i.p., once every two days) Anti-PD-1 RMP1-14 (200 $\mu$g/mouse, i.p., once every three days) |
| 5. Lenvatinib & Anti-PD-1 (n=10) | Lenvatinib (10 mg/kg, p.o., once a day) Anti-PD-1 RMP1-14 (200 $\mu$g/mouse, i.p., once every three days) |
| 6. Hydroxyprogesterone Caproate, Lenvatinib & Anti-PD-1 (n=10) | Hydroxyprogesterone Caproate (20 mg/kg, i.p., once every two days) Lenvatinib (10 mg/kg, p.o., once a day) Anti-PD-1 RMP1-14 (200 $\mu$g/mouse, i.p., once every three days) |

**Efficacy Evaluation**

Observations:

Throughout the experimental procedure, general conditions and the nervous system of mice were observed and recorded.

Weight and Food Intake:

Weight and food intake were measured twice a week.

Tumor Volume:

Tumor size was measured using calipers twice a week for two-dimensional assessment. Tumor volume was calculated using the formula $V = (X2Y)/2$, where X and Y represent the shorter and longer diameters of the tumor, respectively, expressed in mm3.
Tumor Growth Inhibition (TGI, %) = (Tumor volume of control group - Tumor volume of treatment group) / (Tumor volume of control group) * 100%

Cytokine Detection:

Cytokines (IL-2, IL-4, IL-8, TGF-$\beta$, IFN-$\gamma$) in plasma samples were assessed following the relevant standard operating procedures and instructions provided with assay kits. IFN-$\gamma$ in frozen tumor samples was quantified using the Mouse IFN-$\gamma$ Immunoassay Quantikine ELISA Kit from R&D Systems. IL-2 and TNF-$\alpha$ in tumor lysate samples were analyzed quantitatively using the Proinflammatory Panel 1 (mouse) Kit from Meso Scale Diagnostics.

Assessment of Cancer Cell Viability and Proliferation:

Immunohistochemical (IHC) analysis was conducted using anti-KI67 antibodies, serving as proliferation markers for assessing tumor cell growth fractions.

Evaluation of CD8+ T Cell Infiltration at Tumor Sites: IHC analysis was performed using anti-CD8 antibodies.

Evaluation of CD4+ T Cells: IHC analysis was conducted using anti-CD4 antibodies.

Evaluation of Natural Killer Cells: IHC analysis was performed using anti-CD335 antibodies.

Microvessel Density Assessment using CD31 Staining: Evaluation of tumor microvessel density was conducted using CD31 staining.

Detection of IFN-$\gamma$ Signaling Pathways in Tumor Tissues.

**Results**

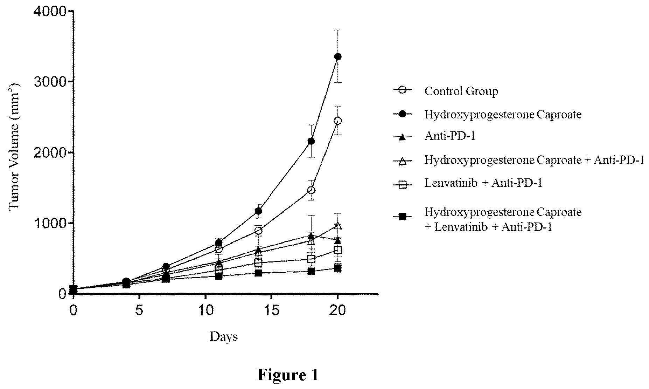

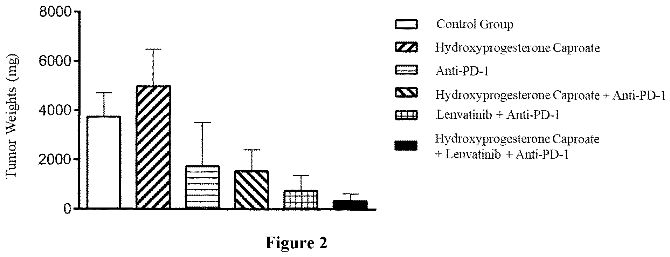

1. The changes in tumor volume due to combination therapy are depicted in Figure 1, while the tumor weights at the conclusion of the combined therapy experiment are illustrated in Figure 2. The tumor inhibition rates are presented in Table 1.

Table 1 Tumor Inhibition Rate (Compared with Control Group)

| Group | Experiment Days | | | | | | |
|---|---|---|---|---|---|---|---|
| | Day 0 | Day 4 | Day 7 | Day 11 | Day 14 | Day 18 | Day 20 |
| HPC | 0.00% | 3.66% | -14.89% | -13.61% | -31.32% | -47.15% | -37.09% |
| Anti-PD-1 | 0.16% | 13.80% | 10.76% | 27.90% | 28.75% | 43.49% | 68.97% |
| HPC + Anti-PD-1 | 0.17% | 7.88% | 19.99% | 31.70% | 34.29% | 48.85% | 60.45% |
| Lenvatinib + Anti-PD-1 | 0.16% | 10.86% | 35.27% | 47.54% | 50.51% | 66.38% | 74.56% |
| HPC + Lenvatinib + Anti-PD-1 | 0.03% | 26.06% | 39.20% | 60.54% | 66.91% | 78.17% | 85.10% |

The results from Figures 1 and 2, as well as Table 1, indicate that the sole administration of Hydroxyprogesterone Caproate did not exhibit a favorable tumor suppression effect in the BALB/c mouse CT26 tumor model. In fact, it demonstrated an adverse effect by promoting tumor growth, leading to an increase in tumor volume. However, co-administration with Hydroxyprogesterone Caproate further enhanced the inhibitory effects of Lenvatinib and anti-PD-1 antibody on the growth of BALB/c mouse CT26 tumors.

2. Co-administration with Hydroxyprogesterone Caproate further improves the regulatory effect of Lenvatinib and anti-PD-1 antibody on the immunosuppressive microenvironment in BALB/c mouse CT26 tumors.

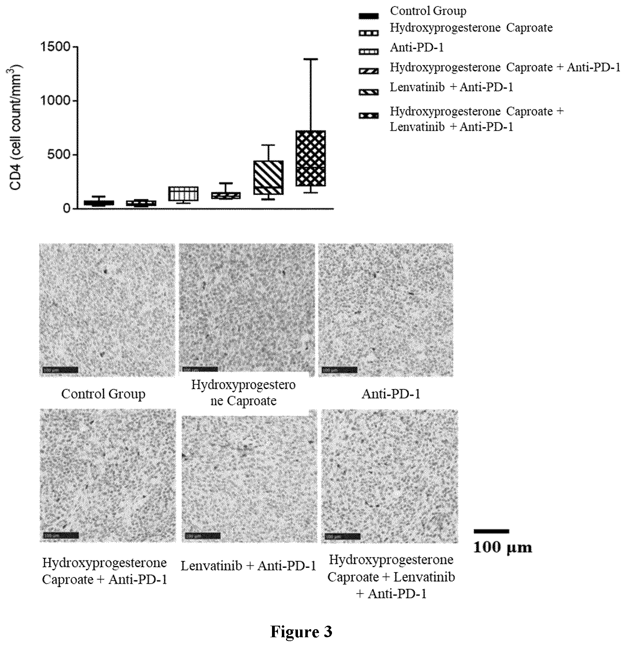

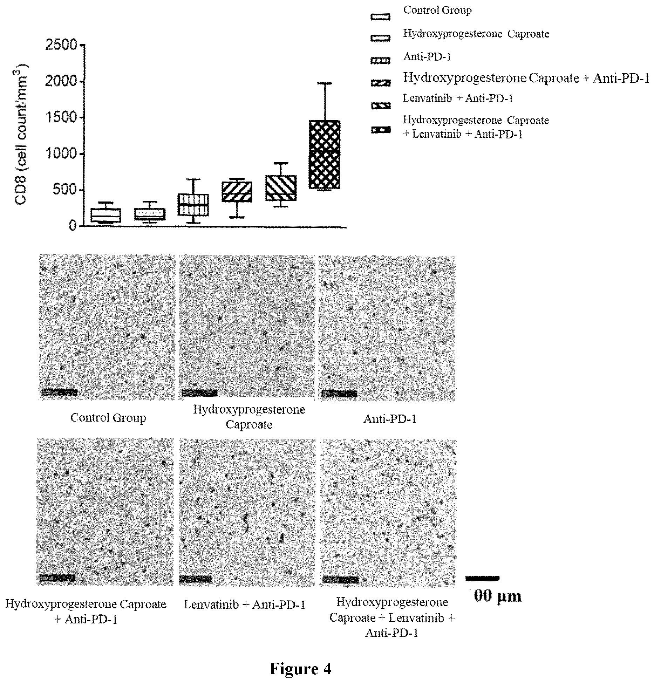

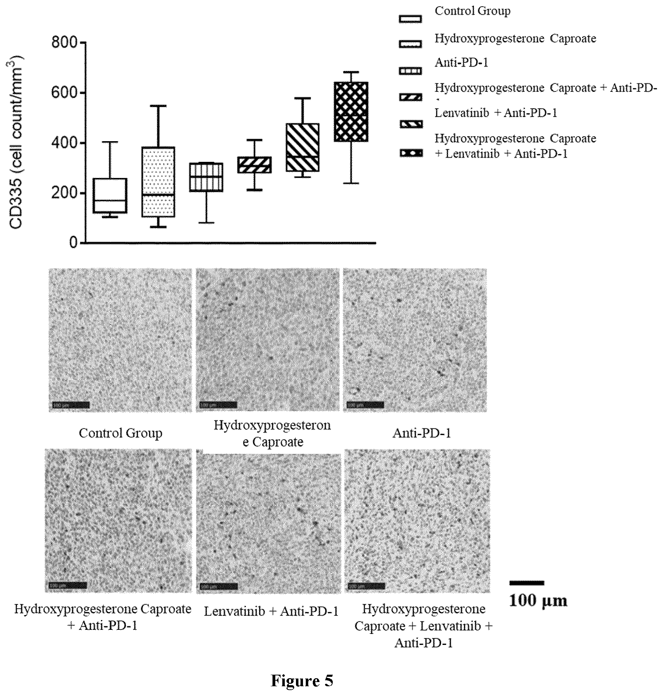

The infiltration effects of CD4+ T cells, CD8+ T cells, and natural killer cells in tumor tissues are shown in Figures 3, 4, and 5, respectively. Figures 3 to 5 demonstrate that in the CT26 colorectal cancer model in mice, co-administration of Hydroxyprogesterone Caproate with Lenvatinib and anti-PD-1 antibody significantly enhances the infiltration of immune cells, including CD4+ T cells, CD8+ T cells, and natural killer cells in tumor tissues, compared to the group treated with Lenvatinib and anti-PD-1 antibody alone.

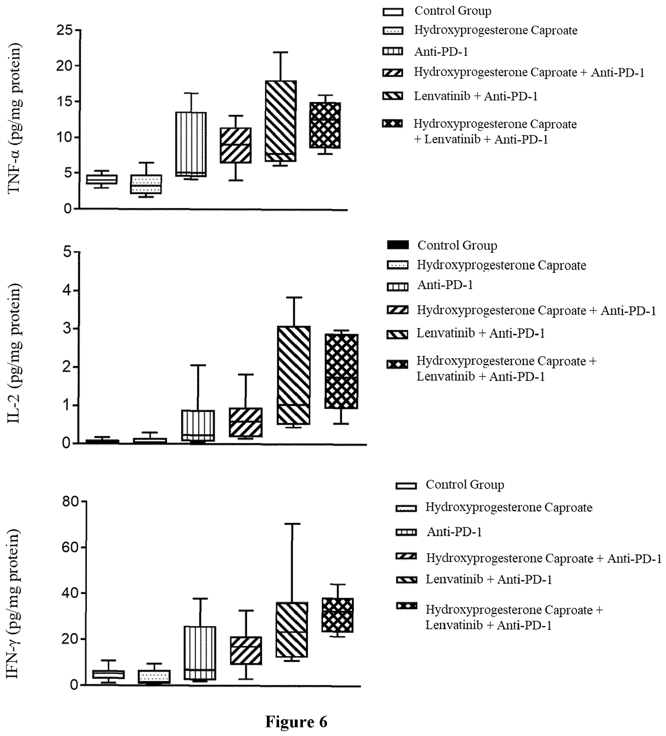

Simultaneously, the triple-drug combination significantly increases the levels of cytokines with tumor-suppressive effects, including TNF-$\alpha$, IL-2, and IFN-$\gamma$, as depicted in Figure 6.

These results indicate that the combination of Hydroxyprogesterone Caproate with Lenvatinib and anti-PD-1 antibody plays a regulatory role in the immunosuppressive microenvironment of tumors.

3. Co-administration with Hydroxyprogesterone Caproate further enhances the regulatory effect of Lenvatinib and anti-PD-1 antibody on angiogenesis in BALB/c mouse CT26 tumors.

**Example 3: Triple Combination of Hydroxyprogesterone Caproate, Lenvatinib, and Pembrolizumab**

**Objective:**

To compare the anti-tumor efficacy of the triple combination of Hydroxyprogesterone Caproate, Lenvatinib, and Pembrolizumab with the combination of Lenvatinib and Pembrolizumab in a humanized PD-1 mouse MC38/hPD-L1 tumor model.

**Study Design:**

1. Establishment of the MC38/hPD-L1 Tumor Model in Humanized PD-1 Mice

Humanized PD-1 mice, female, aged 6-8 weeks, weighing approximately 20 grams, were used. MC38/hPD-L1 cells ($5.0\times106$) dissolved in $100\mu L$ PBS were injected subcutaneously into the humanized PD-1 mice. Upon reaching a tumor volume of approximately ~100 mm3, experimental animals were randomly assigned to control and various treatment groups and subsequently initiated with drug administration.

Lenvatinib (Eisai, Japan) was administered orally once daily at a dose of 10 mg/kg. Hydroxyprogesterone Caproate was administered via intraperitoneal injection every two days. Pembrolizumab was administered every three days at a dose of 200 $\mu$g per mouse. The duration of drug administration lasted around 20 days.

2. Administration Group

| Group | Drug Administration |
|---|---|
| 1. Control group (n=10) | Control agents |
| 2. Hydroxyprogesterone Caproate (n=10) | Hydroxyprogesterone Caproate (20 mg/kg, i.p., once every two days) |
| 3. Pembrolizumab (n=10) | Pembrolizumab (200 $\mu$g/mouse, i.p., once every three days) |
| 4. Hydroxyprogesterone Caproate & Pembrolizumab (n=10) | Hydroxyprogesterone Caproate (20 mg/kg, i.p., once every two days) Pembrolizumab (200 $\mu$g/mouse, i.p., once every three days) |
| 5. Lenvatinib & Pembrolizumab (n=10) | Lenvatinib (10 mg/kg, p.o., once a day) Pembrolizumab (200 $\mu$g/mouse, i.p., once every three days) |
| 6. Hydroxyprogesterone Caproate, Lenvatinib & Pembrolizumab (n=10) | Hydroxyprogesterone Caproate (20 mg/kg, i.p., once every two days) Lenvatinib (10 mg/kg, p.o., once a day) Pembrolizumab (200 $\mu$g/mouse, i.p., once every three days) |

3. Efficacy Evaluation

1) Assessment of mouse body weight and general physical condition during drug administration.
2) Tumor volume measured twice weekly. Calculation method for tumor volume:

$$\text{Tumor Volume (mm}^3) = \text{Length} \times \text{Width}^2/2;$$

3) Evaluation of the effect of CD8+ T cell infiltration at tumor sites.
4) Determination of tumor microvascular density through CD31 staining.
5) Detection of IFN-$\gamma$ signaling within tumor tissues.

Results

Monotherapy with Hydroxyprogesterone Caproate did not exhibit significant tumor inhibitory effects in the humanized PD-1 mouse MC38/hPD-L1 tumor model. However, combination therapy with Hydroxyprogesterone Caproate further enhanced the anti-tumor effects of Lenvatinib and Pembrolizumab in the humanized PD-1 mouse MC38/hPD-L1 tumor model.

**Example 4: Hydroxyprogesterone Caproate, Lenvatinib, and Anti-PD-L1 Antibody (10F.9G2) Triple Combination**

**Objective**

To compare the anti-tumor efficacy of the triple combination of Hydroxyprogesterone Caproate, Lenvatinib, and Anti-PD-L1 Antibody (10F.9G2) with the dual combination of Lenvatinib and Anti-PD-L1 Antibody (10F.9G2) in the CT26 tumor model of BALB/c mice.

**Study Design**

1. Establishment of the BALB/c Mouse CT26 Tumor Model

Female BALB/c mice aged 6-8 weeks, weighing approximately 20 grams, were used. CT26 cells ($5.0\times106$) dissolved in 100 $\mu$L PBS were subcutaneously injected into the BALB/c mice. When the tumor volume reached ~100 mm3, the experimental animals were randomly assigned to control and treatment groups, and drug administration commenced.
Lenvatinib (Eisai, Japan) was administered orally once daily at a dose of 10 mg/kg; Hydroxyprogesterone Caproate was administered via intraperitoneal injection every two days; 10F.9G2 was administered every three days at a dose of 200 $\mu$g/mouse. Drug administration continued for approximately 20 days.

2. Administration Group

| Group | Administration |
| --- | --- |
| 1. Control group (n=10) | Control agents |
| 2. Hydroxyprogesterone Caproate (n=10) | Hydroxyprogesterone Caproate (20 mg/kg, i.p., once every two days) |
| 4. Hydroxyprogesterone Caproate & 10F.9G2 (n=10) | Hydroxyprogesterone Caproate (20 mg/kg, i.p., once every two days) <br> 10F.9G2 (200 $\mu$g/mouse, i.p., once every three days) |
| 5. Lenvatinib & 10F.9G2 (n=10) | Lenvatinib (10 mg/kg, p.o., once a day) <br> 10F.9G2 (200 $\mu$g/mouse, i.p., once every three days) |
| 6. Hydroxyprogesterone Caproate, Lenvatinib & 10F. 9G2 (n=10) | Hydroxyprogesterone Caproate (20 mg/kg, i.p., once every two days) <br> Lenvatinib (10 mg/kg, p.o., once a day) <br> 10F.9G2 (200 $\mu$g/mouse, i.p., once every three days) |

3. Efficacy Evaluation

1) During drug administration, the mice were assessed for their body weight and general physical condition.
2) Tumor volume was measured twice a week, and its size was calculated using the formula:

$$\text{Tumor volume (mm}^3) = \text{length} \times \text{width}^2/2.$$

3) Evaluate the infiltration of CD8+ T cells into the tumor site.
4) Determine tumor micro-vessel density through CD31 staining.
5) Assess the IFN-$\gamma$ signal within the tumor tissue.

**Results**

Hydroxyprogesterone Caproate alone did not exhibit significant tumor suppression in the BALB/c mouse CT26 tumor model. However, the combination therapy involving Hydroxyprogesterone Caproate enhanced the anti-tumor effects of Lenvatinib and Anti-PD-L1 Antibody (10F.9G2) in the BALB/c mouse CT26 tumor model.

**Example 5: Combined Use of Different Progestins with Lenvatinib and RMP1-14**

**Object**

To compare the therapeutic effects of various progestins (hydroxyprogesterone caproate, medroxyprogesterone acetate, norethisterone, progesterone) in conjunction with Lenvatinib and RMP1-14 (anti-PD-1 antibody) in treating CT26 tumor models in BALB/c mice.

**Study Design**

1. Establishment of BALB/c Mouse CT26 Tumor Model:

Female BALB/c mice aged 6-8 weeks, weighing approximately 20 grams, received subcutaneous injections of CT26 cells ($5.0 \times 10^6$) dissolved in 100 μL of PBS. Once tumors reached a volume of approximately ~100 mm3, mice were randomly divided into control and treatment groups for drug administration.
Lenvatinib was orally administered daily at a dosage of 10 mg/kg. The various progestins were administered via intraperitoneal injections every two days. Anti-PD-1 antibody (RMP1-14) was diluted in PBS and administered every three days at a dosage of 200 μg per mouse. Drug administration continued for about 20 days.

2. Administration Group

| Group | Administration |
|---|---|
| 1. Control group (n=10) | Control agents |
| 2. Lenvatinib & RMP1-14(n=10) | Lenvatinib (10 mg/kg, p.o., once a day)<br>RMP1-14 (200 μg/mouse, i.p., once every three day) |
| 3. Hydroxyprogesterone Caproate, Lenvatinib & RMP1-14(n=10) | Hydroxyprogesterone Caproate (20 mg/kg, i.p., once every two day)<br>Lenvatinib (10 mg/kg, p.o., once a day)<br>RMP1-14 (200 μg/mouse, i.p., once every three day) |
| 4. Medroxyprogesterone Acetate, Lenvatinib & RMP1-14(n=10) | Medroxyprogesterone Acetate (20 mg/kg, i.p., once every two day)<br>Lenvatinib (10 mg/kg, p.o., once a day)<br>RMP1-14 (200 μg/mouse, i.p., once every three day) |
| 5. Norethisterone, Lenvatinib & RMP1-14(n=10) | Norethisterone (20 mg/kg, i.p., once every two day)<br>Lenvatinib (10 mg/kg, p.o., once a day)<br>RMP1-14 (200 μg/mouse, i.p., once every three day) |
| 6. Progesterone, Lenvatinib & RMP1-14(n=10) | Progesterone (20 mg/kg, i.p., once every two day)<br>Lenvatinib (10 mg/kg, p.o., once a day)<br>RMP1-14 (200 μg/mouse, i.p., once every three day) |

3. Efficacy Evaluation

1) During drug administration, the mice were assessed for their body weight and general physical condition.

2) Tumor volume was measured twice a week, and its size was calculated using the formula:

$$\text{Tumor volume (mm}^3) = \text{length} \times \text{width}^2/2.$$

**Results**

Compared to other types of progestins, hydroxyprogesterone caproate significantly enhances the anti-tumor effects of Lenvatinib and anti-PD-1 antibody in the BALB/c mouse CT26 tumor model.

**Example 6 Renca Renal Cell Carcinoma Model and Drug Administration**

**Object**

To compare the anti-tumor efficacy between the combination of hydroxyprogesterone caproate, Lenvatinib, and anti-PD-1 antibody and the combination of Lenvatinib and anti-PD-1 antibody in the BALB/c mouse model of Renca renal cell carcinoma.

**Study Design**

1. Establishment of the Renca Tumor Model in BALB/c Mice

BALB/c female mice, aged 6-8 weeks and weighing around 20 grams, were utilized. Renca cells ($3.0 \times 106$) dissolved in 100 $\mu$L PBS were subcutaneously injected into the BALB/c mice. Upon reaching a tumor volume of about 70 mm3, experimental animals were randomly distributed into control and treatment groups.

Lenvatinib was administered orally once daily at a dosage of 7.5 mg/kg; hydroxyprogesterone caproate was administered intraperitoneally (abbreviated as i.p. in the table) every two days. Anti-PD-1 antibody (BioXCell, Supplier: Yicon (Beijing) Medical Technology Inc.) was diluted in PBS and administered every four days at a dose of 100 $\mu$g per mouse.

2. Administration Group

| Group | Administration |
|---|---|
| 1. Control group (n=10) | Control agents |
| 2. Hydroxyprogesterone Caproate (n=10) | Hydroxyprogesterone Caproate (20 mg/kg, i.p., once every two days) |
| 3. Anti-PD-1 (n=10) | Anti-PD-1 (100 $\mu$g/mouse, i.p., once every four days) |
| 4. Lenvatinib (n=10) | Lenvatinib (7.5 mg/kg, p.o., once a day) |
| 5. Hydroxyprogesterone Caproate & Anti-PD-1 (n=10) | Hydroxyprogesterone Caproate (20 mg/kg, i.p., once every two days) Anti-PD-1 (100 $\mu$g/mouse, i.p., once every four days) |
| 6. Lenvatinib & Anti-PD-1 (n=10) | Lenvatinib (7.5 mg/kg, p.o., once a day) Anti-PD-1 (100 $\mu$g/mouse, i.p., once every four days) |
| 7. Hydroxyprogesterone Caproate, Lenvatinib & Anti-PD-1 (n=10) | Hydroxyprogesterone Caproate (20 mg/kg, i.p., once every two days) Lenvatinib (7.5 mg/kg, p.o., once a day) Anti-PD-1 (100 $\mu$g/mouse, i.p., once every four days) |

3. Efficacy Evaluation

Observation: Throughout the experiment, general conditions and the nervous system of the mice were observed and recorded.

Weight and Food Intake: Weight and food intake were measured twice a week.

Tumor Volume:

Tumor size was measured biweekly using calipers for two-dimensional measurements. The volume was calculated using the formula and expressed in mm$^3$: $V = (X^2Y)/2$, where X and Y represent the shorter and longer diameters of the tumor, respectively.

$$\text{Tumor Growth Inhibition Rate (TGI, \%)} = (\text{Tumor volume of control group} -$$

$$\text{Tumor volume of treatment group}) / (\text{Tumor volume of control group}) * 100\%$$

**Results**

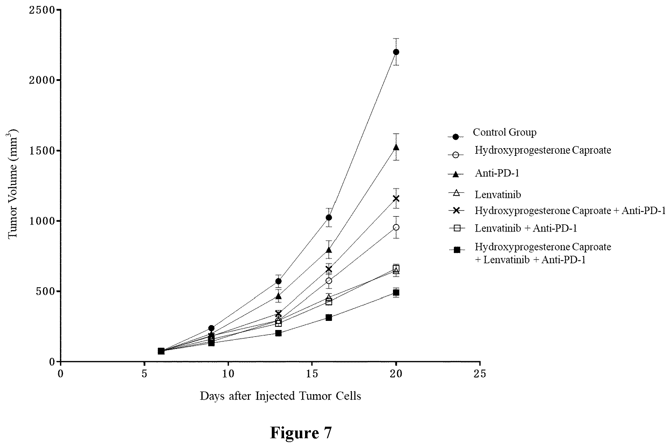

The change in tumor volume for each treatment group is illustrated in Figure 7, and the tumor inhibition rates are presented in Table 2.

Table 2 Tumor Inhibition Rates (Compared with Control Group)

| Group | Experiment Day | | | | |
|---|---|---|---|---|---|
| | Day 6 | Day 9 | Day 13 | Day 16 | Day 20 |
| Hydroxyprogesterone Caproate | 0.00% | 40.00% | 48.51% | 43.95% | 56.59% |
| Anti-PD-1 | 0.00% | 16.67% | 18.04% | 22.27% | 30.68% |
| Lenvatinib | 0.00% | 22.92% | 48.51% | 55.37% | 70.68% |
| Hydroxyprogesterone Caproate + Anti-PD-1 | 0.00% | 24.17% | 39.93% | 35.74% | 47.27% |
| Lenvatinib + Anti-PD-1 | 0.00% | 32.92% | 52.19% | 58.30% | 69.91% |
| Hydroxyprogesterone Caproate + Lenvatinib + Anti-PD-1 | -1.32% | 44.17% | 64.27% | 69.24% | 77.73% |

The results from Figure 7 and Table 2 show that:
Mono-treatment with hydroxyprogesterone caproate exhibits a certain degree of tumor suppression in the BALB/c mouse Renca tumor model, with a tumor inhibition rate higher than that observed with monotherapy using anti-PD-1 antibodies alone. Co-administration with hydroxyprogesterone caproate further enhances the inhibitory effects of lenvatinib and anti-PD-1 antibodies on the growth of Renca tumors in BALB/c mice.

**Claims**

1. A method for inhibiting tumors in a subject, **characterized by** administering hydroxyprogesterone caproate, along with receptor tyrosine kinase inhibitors and/or anti-PD-1/PD-L1 antibodies to the subject.

2. The use of hydroxyprogesterone caproate, along with receptor tyrosine kinase inhibitors and/or anti-PD-1/PD-L1 antibodies in the preparation of drugs for treating tumors.

3. A drug for treating tumors, comprising hydroxyprogesterone caproate, along with receptor tyrosine kinase inhibitors and/or anti-PD-1/PD-L1 antibodies.

4. The method, drug, or use according to any one of claims 1-3, wherein the tumor is colorectal, endometrial, hepatocellular, melanoma, non-small cell lung, renal cell, head and neck squamous cell, urothelial, biliary tract, colorectal, gastric, glioblastoma, ovarian, pancreatic, or triple-negative breast cancer.

5. The method, drug, or use according to claim 4, wherein the colorectal tumor is colon tumor, preferably formed by CT26 tumor cells.

6. The method, drug, or use according to any one of claims 1-5, wherein the dosage of hydroxyprogesterone caproate is 1-100 mg/kg.

7. The method, drug, or use according to claim 6, wherein the dosage of hydroxyprogesterone caproate is 5-80 mg/kg.

8. The method, drug, or use according to claim 7, wherein the dosage of hydroxyprogesterone caproate is 10-70 mg/kg.

9. The method, drug, or use according to claim 8, wherein the dosage of hydroxyprogesterone caproate is 20-60 mg/kg.

10. The method, drug, or use according to claim 9, wherein the dosage of hydroxyprogesterone caproate is 20-40 mg/kg.

11. The method, drug, or use according to claim 10, wherein the dosage of hydroxyprogesterone caproate is 20 mg/kg.

12. The method, drug, or use according to any one of claims 1-11, wherein the frequency of hydroxyprogesterone caproate administration is from twice daily to once every 5 days.

13. The method, drug, or use according to claim 12, wherein the frequency of hydroxyprogesterone caproate administration is from once daily to once every 3 days.

14. The method, drug, or use according to claim 13, wherein the frequency of hydroxyprogesterone caproate administration is once every 2 days.

15. The method, drug, or use according to any one of claims 1-14, wherein the dosage of the receptor tyrosine kinase inhibitor is 1-20 mg/kg.

16. The method, drug, or use according to claim 15, wherein the dosage of the receptor tyrosine kinase inhibitor is 5-15 mg/kg.

17. The method, drug, or use according to claim 16, wherein the dosage of the receptor tyrosine kinase inhibitor is 10 mg/kg.

18. The method, drug, or use according to any one of claims 1-17, wherein the frequency of receptor tyrosine kinase inhibitor administration is from twice daily to once every 2 days.

19. The method, drug, or use according to claim 18, wherein the frequency of receptor tyrosine kinase inhibitor administration is once daily.

20. The method, drug, or use according to any one of claims 1-19, wherein the dosage of anti-PD-1/PD-L1 antibodies is 0.1-10 mg/kg.

21. The method, drug, or use according to claim 20, wherein the dosage of anti-PD-1/PD-L1 antibodies is 0.5-5 mg/kg.

22. The method, drug, or use according to claim 21, wherein the dosage of anti-PD-1/PD-L1 antibodies is 1-3 mg/kg.

23. The method, drug, or use according to claim 22, wherein the dosage of anti-PD-1/PD-L1 antibodies is 2 mg/kg.

24. The method, drug, or use according to any one of claims 1-23, wherein the frequency of anti-PD-1/PD-L1 antibody administration is from twice daily to once every 6 days.

25. The method, drug, or use according to claim 24, wherein the frequency of anti-PD-1/PD-L1 antibody administration is from once daily to once every 5 days.

26. The method, drug, or use according to claim 25, wherein the frequency of anti-PD-1/PD-L1 antibody administration is once every 3 days.

27. The method, drug, or use according to any one of claims 1-26, wherein the receptor tyrosine kinase inhibitor is avapritinib, capmatinib, pemigatinib, ripretinib, selpercatinib, selumetinib, tucatinib, entrectinib, erdafitinib, fedratinib, pexidartinib, upadacitinib, zanubrutinib, baricitinib, binimetinib, dacomitinib, fostamatinib, gilteritinib, larotrectinib, lorlatinib, acalabrutinib, brigatinib, midostaurin, neratinib, alectinib, cobimetinib, lenvatinib, osimertinib, ceritinib, nintedanib, afatinib, ibrutinib, trametinib, axitinib, bosutinib, cabozantinib, ponatinib, regorafenib, tofacitinib, crizotinib, ruxolitinib, vandetanib, pazopanib, lapatinib, nilotinib, dasatinib, sunitinib, sorafenib, erlotinib, gefitinib, and/or imatinib; preferably lenvatinib.

28. The method, drug, or use according to any one of claims 1-27, wherein the anti-PD-1 antibody/PD-L1 is pamulimab, 10F.9G2, RMP1-14, RMP1-1, navolimab, cimiplimab, tremelimumab, sintilimab, teplizumab, atezolizumab, avelumab, ceralifimab, tiragolumab, dostarlimab, dotatate, and/or ravulizumab.

29. The method, drug, or use according to any one of claims 1-28, wherein hydroxyprogesterone caproate, receptor tyrosine kinase inhibitors, and anti-PD-1/PD-L1 antibodies are in the form of oral or injectable preparations.

30. The drug or use according to claims 2-29, wherein the proportions of hydroxyprogesterone caproate, receptor tyrosine kinase inhibitors, and anti-PD-1/PD-L1 antibodies in the drug are 1-100:2-20:0.1-10.

31. The drug or use according to claim 30, wherein the proportions of hydroxyprogesterone caproate, receptor tyrosine kinase inhibitors, and anti-PD-1/PD-L1 antibodies in the drug are 20-60:5-15:0.5-5.

32. The drug or use according to claim 31, wherein the proportions of hydroxyprogesterone caproate, receptor tyrosine kinase inhibitors, and anti-PD-1/PD-L1 antibodies in the drug are 20:10:2.

33. The application of hydroxyprogesterone caproate in enhancing the effects of receptor tyrosine kinase inhibitors and/or anti-PD-1/PD-L1 antibodies.

34. The application of hydroxyprogesterone caproate in the preparation of drugs to enhance the effects of receptor tyrosine kinase inhibitors and/or anti-PD-1/PD-L1 antibodies.

35. The application according to claim 33 or 34, wherein the effects include inhibiting tumor growth.

36. The application according to claim 33 or 34, wherein the effects include improving the regulation of the tumor immune-suppressive microenvironment.

37. The application according to claim 33 or 34, wherein the effects include enhancing infiltration of CD8+ T cells.

38. The application according to claim 33 or 34, wherein the effects include improving the regulation of tumor angiogenesis.

39. The application according to any one of claims 33-38, wherein the tumor is endometrial or colorectal.

40. The application according to claim 39, wherein the colorectal tumor is colon tumor, preferably formed by CT26 tumor cells.

41. The application according to any one of claims 33-40, wherein the dosage of hydroxyprogesterone caproate is 1-100 mg/kg.

42. The application according to claim 41, wherein the dosage of hydroxyprogesterone caproate is 5-80 mg/kg.

43. The application according to claim 42, wherein the dosage of hydroxyprogesterone caproate is 10-70 mg/kg.

44. The application according to claim 43, wherein the dosage of hydroxyprogesterone caproate is 20-60 mg/kg.

45. The application according to claim 44, wherein the dosage of hydroxyprogesterone caproate is 20-40 mg/kg.

46. The application according to claim 45, wherein the dosage of hydroxyprogesterone caproate is 20 mg/kg.

47. The application according to any one of claims 33-46, wherein the frequency of hydroxyprogesterone caproate administration is from twice daily to once every 5 days.

**48.** The application according to claim 47, wherein the frequency of hydroxyprogesterone caproate administration is from once daily to once every 3 days.

**49.** The application according to claim 48, wherein the frequency of hydroxyprogesterone caproate administration is once every 2 days.

**50.** The application according to any one of claims 33-49, wherein the receptor tyrosine kinase inhibitor is lenvatinib.

**51.** The application according to any one of claims 34-50, wherein the anti-PD-1/PD-L1 antibody is pamulimab, 10F.9G2, RMP1-14, RMP1-1, navolimab, cimiplimab, tremelimumab, sintilimab, teplizumab, atezolizumab, avelumab, ceralifimab, tiragolumab, dostarlimab, dotatate, and/or ravulizumab.

**52.** The application according to any one of claims 34-51, wherein hydroxyprogesterone caproate is in the form of oral or injectable preparations.

**Figure 1**

**Figure 2**

Figure 3

Figure 4

Figure 5

**Figure 6**

Figure 7

## INTERNATIONAL SEARCH REPORT

| International application No. |
|---|
| **PCT/CN2022/098284** |

**A. CLASSIFICATION OF SUBJECT MATTER**

A61K 31/57(2006.01)i; A61K 45/06(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNPAT, CNKI, DWPI, SIPOABS, CAPLUS, REGISTRY: 深圳埃格林医药有限公司, 杜涛, 胡涛, 杜新, 张玉倩, 己酸羟孕酮, 酪氨酸激酶抑制剂, 仑伐替尼, 乐伐替尼, 抗PD-1 抗体, 抗PD-L1抗体, 派姆单抗, 缺氧诱导因子, 17-HPC, hydroxyprogesterone caproate, 630-56-8, tyrosine kinase inhibitor, lenvatinib, PD-1, PD-L1, anti-PD-1 antibody, anti-PD-L1 antibody, pembrolizumab, hypoxia inducible factor, HIF

**C. DOCUMENTS CONSIDERED TO BE RELEVANT**

| Category* | Citation of document, with indication, where appropriate, of the relevant passages | Relevant to claim No. |
|---|---|---|
| PY | WO 2021262562 A2 (MERCK SHARP & DOHME et al.) 30 December 2021 (2021-12-30) claims 1 and 25 | 1-52 |
| PY | WO 2021262563 A1 (MERCK SHARP & DOHME et al.) 30 December 2021 (2021-12-30) claim 1 | 1-52 |
| Y | CN 108159058 A (SHANGHAI LIPU BIOMEDICINE TECH CO., LTD.) 15 June 2018 (2018-06-15) description paragraphs [0007], [0009], [0020], and [0023] | 1-52 |
| Y | US 2018092901 A1 (MERCK SHARP & DOHME et al.) 05 April 2018 (2018-04-05) claims 1 and 9 | 1-52 |
| Y | US 2018140569 A1 (PELOTON THERAPEUTICS INC) 24 May 2018 (2018-05-24) claims 1, 3, 45, and 46, and description paragraph [0003] | 1-52 |

☐ Further documents are listed in the continuation of Box C.   ☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **25 August 2022** | **15 September 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2022/098284**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **1、4-29、33、35-52**
   because they relate to subject matter not required to be searched by this Authority, namely:

   [1] These claims relate to a method for treatment of a disease (PCT Rule 39.1(iv)); however, a search was still conducted for a corresponding pharmaceutical use of the substance.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.

**PCT/CN2022/098284**

| Patent document cited in search report | | | Publication date (day/month/year) | Patent family member(s) | | | Publication date (day/month/year) |
|---|---|---|---|---|---|---|---|
| WO | 2021262562 | A2 | 30 December 2021 | None | | | |
| WO | 2021262563 | A1 | 30 December 2021 | None | | | |
| CN | 108159058 | A | 15 June 2018 | None | | | |
| US | 2018092901 | A1 | 05 April 2018 | KR | 20170122809 | A | 06 November 2017 |
| | | | | CA | 2978226 | A1 | 09 September 2016 |
| | | | | US | 2022023285 | A1 | 27 January 2022 |
| | | | | WO | 2016141218 | A1 | 09 September 2016 |
| | | | | WO | 2016140717 | A1 | 09 September 2016 |
| | | | | JP | 2021035962 | A | 04 March 2021 |
| | | | | JP | 2018512391 | A | 17 May 2018 |
| | | | | AU | 2015384801 | A1 | 07 September 2017 |
| US | 2018140569 | A1 | 24 May 2018 | US | 2019282535 | A1 | 19 September 2019 |
| | | | | WO | 2016168510 | A1 | 20 October 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS,* 630-56-8